# EUROPEAN PATENT SPECIFICATION

(11) **EP 3 906 556 B1**
(45) Date of publication and mention of the grant of the patent: **22.04.2026**
(21) Application number: 20735932.4
(22) Date of filing: 02.01.2020
(51) Int. Cl.: G16B 15/30, G16B 40/20, G16B 40/30

(54) **METHOD AND SYSTEM FOR PREDICTING DRUG BINDING USING SYNTHETIC DATA**
VERFAHREN UND SYSTEM ZUR VORHERSAGE DER WIRKSTOFFBINDUNG MITTELS SYNTHETISCHER DATEN
PROCÉDÉ ET SYSTÈME DE PRÉDICTION DE LIAISON DE MÉDICAMENT À L'AIDE DE DONNÉES DE SYNTHÈSE

(30) Priority: 04.01.2019 US 201962788682 P
(43) Date of publication of application: 10.11.2021
(73) Proprietor: Cyclica Inc., Toronto, Ontario M5J 1A7 (CA)
(72) Inventor: MACKINNON, Stephen Scott, Burlington, Ontario L7L 6P1 (CA); SAFIKHANI, Zhaleh, Toronto, Ontario M1R 3R8 (CA); VERNON, Robert, Toronto, Ontario M5M 1K5 (CA); BRERETON, Andrew E., Toronto, Ontario M4V 2V7 (CA); WINDEMUTH, Andreas, Belmont, Massachusetts 02478 (US)
(74) Representative: Guardian IP Consulting I/S
(86) International application number: PCT/CA2020/050005
(87) International publication number: WO 2020/140156

(56) References cited:
- US-A1- 2012 239 367
- US-A1- 2012 239 367
- US-A1- 2014 258 207
- US-B2- 8 949 157
- HUANG YU-AN ET AL: "A Systematic Prediction of Drug-Target Interactions Using Molecular Fingerprints and Protein Sequences", vol. 19, no. 5, 1 May 2018 (2018-05-01), NL, pages 468 - 478, XP055952478, ISSN: 1389-2037, Retrieved from the Internet <URL:https://www.sciencedirect.com/science/article/pii/S1532046419300772/pdfft?md5=50ec83acde1380e5ba7c48de031741ce&pid=1-s2.0-S1532046419300772-main.pdf> [retrieved on 20220817], DOI: 10.2174/1389203718666161122103057
- MEI JIAN-PING ET AL: "Drug-target interaction prediction by learning from local information and neighbors", vol. 29, no. 2, 17 November 2012 (2012-11-17), GB, pages 238 - 245, XP055952094, ISSN: 1367-4803, Retrieved from the Internet <URL:https://academic.oup.com/bioinformatics/article-pdf/29/2/238/17124669/bts670.pdf> [retrieved on 20220816], DOI: 10.1093/bioinformatics/bts670
- FAKHRAEI ET AL.: "Network-Based Drug-Target Interaction Prediction with Probabilistic Soft Logic", IEEE /ACM TRANSACTIONS ON COMPUTATIONAL BIOLOGY AND BIOINFORMATICS, SEPTEMBER, vol. 11, no. 5, September 2014 (2014-09-01), pages 775 - 787, XP011560927, DOI: 10.1109/TCBB.2014.2325031
- YU ET AL.: "A Systematic Prediction of Multiple Drug-Target Interactions from Chemical, Genomic , and Pharmacological Data", PLOS ONE, vol. 7, no. 5, 30 May 2012 (2012-05-30), pages 1 - 14, XP055134306

## Description

### BACKGROUND

Computational methods exist to predict interactions between ligands and proteins. They are generally classified as 'ligand-based' or 'structure-based' in accordance with the type of information used to make the prediction.

Protein-based predictions have the potential to learn biophysical compatibility and therefore may be more universally generalizable but are highly data-bound. Specifically, protein-based predictions use 3D molecular structures of ligands co-crystallized with proteins to evaluate or predict interaction. These methods are computationally demanding, and they tend to be trained on only 100's to 1000's of different proteins. Neural networks trained on these tend to have a very high feature space to data ratio. As a result, the method may produce a significant number of false negatives and/or false positives in docking when applied to previously unseen protein systems or drug scaffolds.

Ligand-based predictions may be performed using Drug Target Interaction (DTI) databases that have millions of records. Examples of publicly-available DTI databases include ChEMBL, NCBI's Bioassay, and STITCH. However, these records tend to only represent ~2,000 of 20,000 human proteins. With high ligand-to-protein data ratios, one standard approach is to derive many different models for each of the 2,000 proteins. These tend to be successful, even beating high-throughput experimental results in many cases, but (1) only represent ~10% of human proteins, (2) may be weaker when there is not much chemical diversity among examples for an individual protein, and (3) individual models do not learn the physical attributes of drug-protein compatibility, missing to benefit from data used to generate other models.
US2012/239367 discloses a method for evaluating a potential interaction between a ligand and a receptor (see Title and Abstract). The method comprises the step of evaluating the potential interaction between the ligand and the receptor based on a predictive model trained using a database. The database describes the affinity with the receptor of a source ligand, and a plurality of additional ligands derived from the source ligand.
US2014/258207 discloses methods for predicting protein-ligand interactions (see Title and Abstract). US2014/258207 teaches that an exemplary method can include identifying a set of structural neighbours of the query proteins, where each of the structural neighbors contains one or more ligands. The method can also determine a similarity score for each of the structural neighbours and predict protein-ligand interactions of the one or more query proteins using the similarity scores.

### SUMMARY OF THE INVENTION

The invention is set out in the appended claims.

### SUMMARY OF RELATED DISCLOSURE

In general, in one aspect, one or more embodiments relate to a method for predicting drug-target binding using synthetically-augmented data, the method comprising: generating a plurality of ghost ligands for a plurality of proteins in a protein structure database; generating a plurality of drug-target interaction (DTI) features for proteins and ligands in a DTI database, using the plurality of ghost ligands; generating a machine learning model using the plurality of DTI features; and predicting a likelihood of interaction for a combination of a query protein and a query ligand using the machine learning model.

In general, in one aspect, one or more embodiments relate to a non-transitory computer readable medium comprising computer readable program code for predicting drug-target binding using synthetically-augmented data, the computer readable program code causing a computer system to: generate a plurality of ghost ligands for a plurality of proteins in a protein structure database; generate a plurality of drug-target interaction (DTI) features for proteins and ligands in a DTI database, using the plurality of ghost ligands; generate a machine learning model using the DTI features; and predict a likelihood of interaction for a combination of a query protein and a query ligand using the machine learning model.

In general, in one aspect, one or more embodiments relate to a system for differential drug discovery, the system comprising: a protein structure database; a ghost ligand identification engine configured to generate a plurality of ghost ligands for a plurality of proteins in the protein structure database; a ghost ligand database storing the plurality of ghost ligands; a drug-target interaction (DTI) database storing proteins and ligand; a feature generation engine configured to generate a plurality of DTI features for the proteins and the ligands in the DTI database, using the plurality of ghost ligands in the ghost ligand database; a machine learning model training engine configured to generate a machine learning model using the DTI features; and a DTI prediction engine configured to predict a likelihood of interaction for a combination of a query protein and a query ligand using the machine learning model.

### BRIEF DESCRIPTION OF DRAWINGS

The present embodiments are illustrated by way of example and are not intended to be limited by the figures of the accompanying drawings.
FIG. 1A shows a block diagram of a system for predicting drug binding in accordance with one or more embodiments.
FIG. 1B shows a block diagram of a protein structure database in accordance with one or more embodiments.
FIG. 1C shows a block diagram of a ghost ligand database in accordance with one or more embodiments.
FIG. 1D shows a block diagram of a drug-target interaction database in accordance with one or more embodiments.
FIG. 1E shows a block diagram of a protein annotation database in accordance with one or more embodiments.
FIG. 2 shows a flowchart describing a method for training a machine learning model for predicting drug-target interactions, in accordance with one or more embodiments.
FIG. 3 shows a flowchart describing a method for generating a ghost ligand database, in accordance with one or more embodiments.
FIG. 4 shows a flowchart describing a method for generating drug-target interaction (DTI) features, in accordance with one or more embodiments.
FIG. 5 shows a flowchart describing a method for generating a machine learning model for DTI prediction, in accordance with one or more embodiments.
FIG. 6 shows a flowchart describing a method for predicting an interaction between a query protein and a query ligand, in accordance with one or more embodiments.
FIG. 7A shows an example for generating ghost ligands, in accordance with one or more embodiments.
FIG. 7B shows an illustration of a concentric shell model used to obtain binding site features, in accordance with one or more embodiments.
FIG. 8 shows the generation of training data for the machine learning model, in accordance with one or more embodiments.
FIG. 9 shows a performance comparison of an embodiment of the disclosure and a conventional approach.
FIG. 10A and FIG. 10B show computing systems, in accordance with one or more embodiments.

### DETAILED DESCRIPTION

Specific embodiments disclosed herein will now be described in detail with reference to the accompanying figures. Like elements in the various figures may be denoted by like reference numerals and/or like names for consistency.

The following detailed description is merely exemplary in nature, and is not intended to limit the embodiments disclosed herein or the application and uses of embodiments disclosed herein. Furthermore, there is no intention to be bound by any expressed or implied theory presented in the preceding technical field, background, brief summary or the following detailed description.

In the following detailed description of some embodiments disclosed herein, numerous specific details are set forth in order to provide a more thorough understanding of the various embodiments disclosed herein. However, it will be apparent to one of ordinary skill in the art that the embodiments may be practiced without these specific details. In other instances, well-known features have not been described in detail to avoid unnecessarily complicating the description.

Throughout the application, ordinal numbers (*e*.*g*., first, second, third, etc.) may be used as an adjective for an element *(i.e.,* any noun in the application). The use of ordinal numbers is not to imply or create any particular ordering of the elements nor to limit any element to being only a single element unless expressly disclosed, such as by the use of the terms "before", "after", "single", and other such terminology. Rather, the use of ordinal numbers is to distinguish between the elements. By way of an example, a first element is distinct from a second element, and the first element may encompass more than one element and succeed (or precede) the second element in an ordering of elements.

In one or more embodiments of the invention, the elements of the protein-based prediction methods and ligand-based prediction methods may be combined to obtain superior predictions of drug-target interactions. In one or more embodiments of the invention, a machine learning model is used to predict drug-target interactions (DTIs).

An abundance of data points exists for protein-ligand interactions in DTI databases. For example, the ChEMBL database contains ~15,000,000 records describing pairs of proteins and ligands as binding or non-binding (and frequently providing a measure of affinity or confidence). However, using the content of these DTI databases to predict interactions of new pairs of proteins and ligands has certain limitations, such as limited coverage of the human proteome and weak predictions when the diversity among examples for an individual protein is limited. Accordingly, the quality of predictions by machine learning models operating purely on DTI database records may be limited. In the alternative, a machine learning model may operate on 3D molecular structures of ligands co-crystallized with proteins to capture the biophysics of interactions between proteins and ligands. A few databases that capture protein-ligand interactions exist (*e.g*., sc-PDB), but these databases contain relatively few data points and tend to be highly redundant. Further, these databases tend to lack protein structure diversity and chemotype diversity. Training a machine learning model on these data points may, thus, be challenging due to an insufficient amount of data. In particular, a machine learning model operating on 3D molecular structures of ligands co-crystallized with proteins has a high-dimensional feature space which, in combination with the limited availability of suitable training samples, makes the machine learning model vulnerable to overfitting.

In one or more embodiments of the disclosure, local 3D features and DTI records are used in combination by a machine learning model to enable superior predictions over the above-described conventional protein-based and ligand-based predictions. More specifically, synthetic data is generated by projecting DTI records onto 3D structure models of known protein-ligand complexes, in order to generate otherwise unavailable local protein features. The synthetic data generated in this manner may be used to train a machine learning model. The machine learning model may then be used to make predictions for pairs of a query protein and a query ligand.

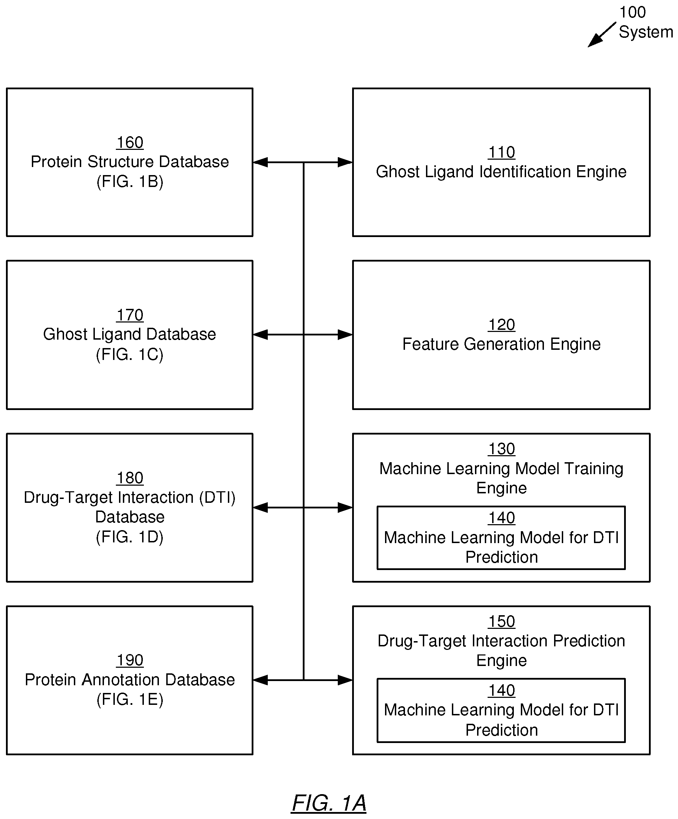

Turning to FIG. 1A, a system for predicting drug binding using synthetic data, in accordance with one or more embodiments, is shown. The system (100) may include a ghost ligand identification engine (110), a feature generation engine (120), a machine learning model training engine (130), a drug-target interaction prediction engine (150), a protein structure database (160), a ghost ligand database (170), a drug-target interaction database (180), and a protein annotation database (190). Each of these components is subsequently described.

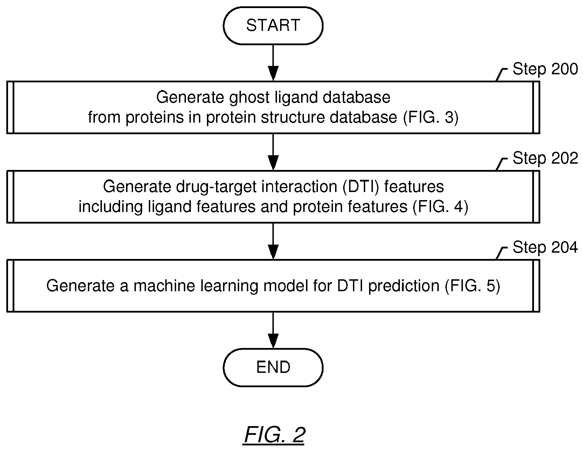

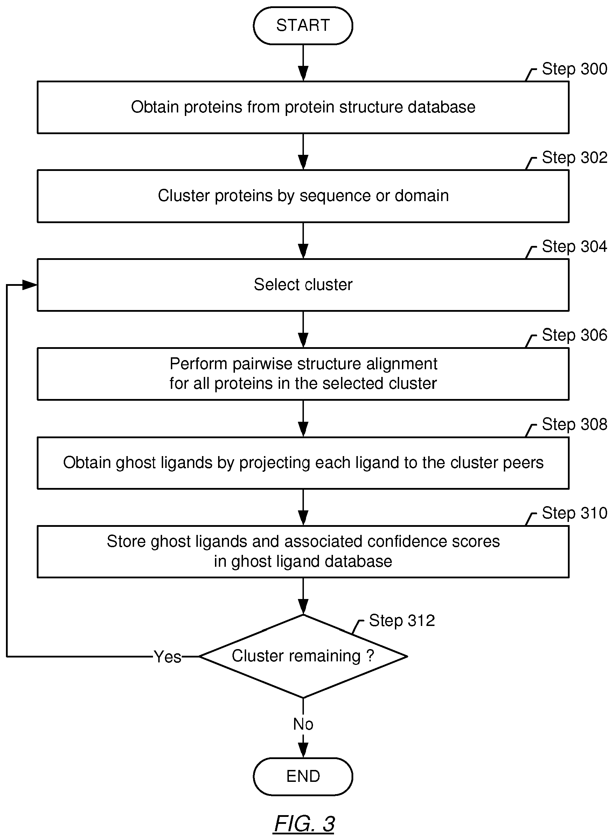

The ghost ligand identification engine (110), in accordance with one or more embodiments, includes instructions in the form of computer readable program code to perform at least one of the steps described in FIGs. 2 and 3 to generate a ghost ligand database (170) of ghost ligands and associated confidence scores from proteins in the protein structure database (160). The ghost ligand identification engine (110) may obtain ghost ligands for a protein by structurally aligning known homologs and by projecting the ligands of these known homologs onto the protein, at the aligned sites. While these ghost ligands may not interact with the protein, they may serve as a placeholder indicating a structural compatibility between the ghost ligands and the aligned sites of the protein. The ghost ligand identification engine (110) is operatively connected to the protein structure database (160) and the ghost ligand database (170).

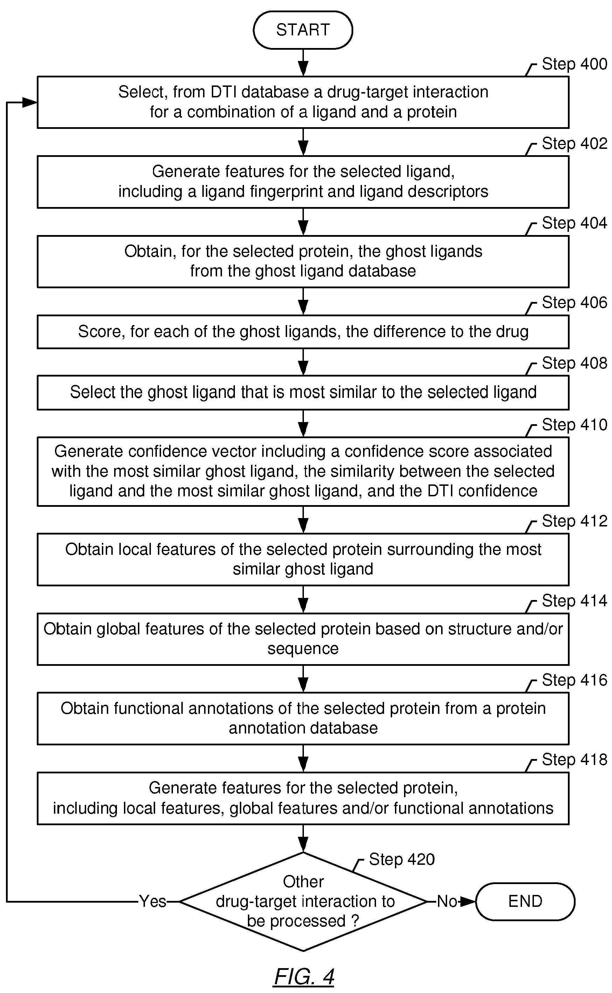

The feature generation engine (120), in accordance with one or more embodiments, includes instructions in the form of computer readable program code to perform at least one of the steps described in FIG. 2 and FIG. 4 to generate drug-target interaction (DTI) features to be used for training a machine learning model for DTI prediction. The feature generation engine (110) may generate features for proteins and ligands using data from the ghost ligand database (170), the drug-target interaction database (180), and the protein annotation database (190). Accordingly, the feature generation engine (120) is operatively connected to the ghost ligand database (170), the drug-target interaction database (180), and the protein annotation database (190).

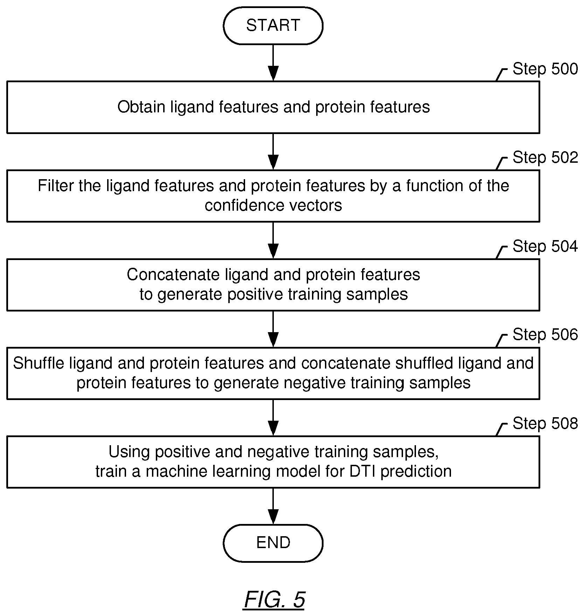

Continuing with FIG. 1 A, the machine learning model training engine (130), in accordance with one or more embodiments, includes instructions in the form of computer readable program code to perform at least one of the steps described in FIG. 2 and FIG. 5 to train a machine learning model (140) for DTI prediction. The machine learning model training engine (130) may use the DTI features generated by the feature generation engine (120) for the training. Accordingly, the machine learning model training engine (130) is operatively connected to the feature generation engine (120). The resulting machine learning model for DTI prediction (140) may be any type of classifier capable of predicting an interaction between a query drug and a query protein. In one or more embodiments, the machine learning model for DTI prediction (140) is a deep neural network.

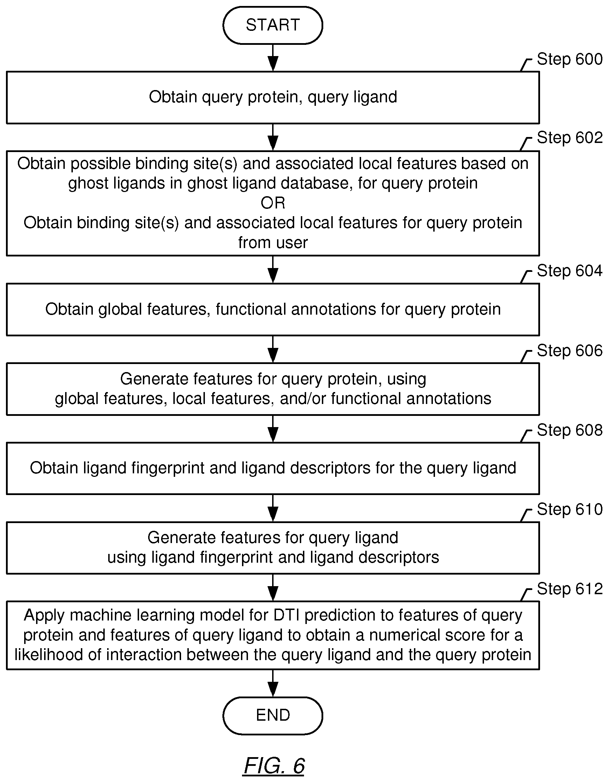

The drug-target interaction (DTI) prediction engine (150), in accordance with one or more embodiments, includes instructions in the form of computer readable program code to perform at least one of the steps described in FIG. 6 to predict drug-target interactions for query drugs and query proteins, using the machine learning model (140). The DTI prediction engine (150) generates features for query proteins and query ligands associated with the query drugs, compatible with the machine learning model (140), and subsequently computes a likelihood of interaction, based on the features, and using the same machine learning model (140) trained by the machine learning model training engine (130). In various embodiments, one or more of the same and/or different machine learning models may be used.

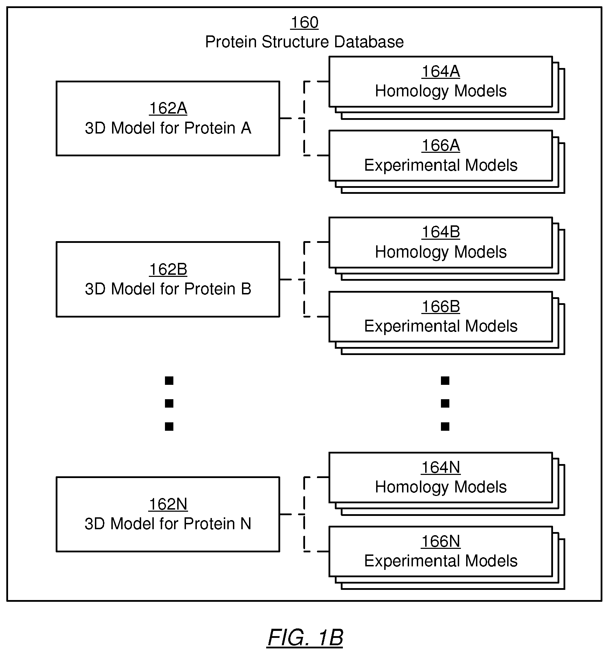

The protein structure database (160), in accordance with one or more embodiments, may be any type of storage unit and/or device (*e.g.,* a file system, database, collection of tables, or any other storage mechanism) for storing data. The protein structure database (160) is described below with reference to FIG. 1B.

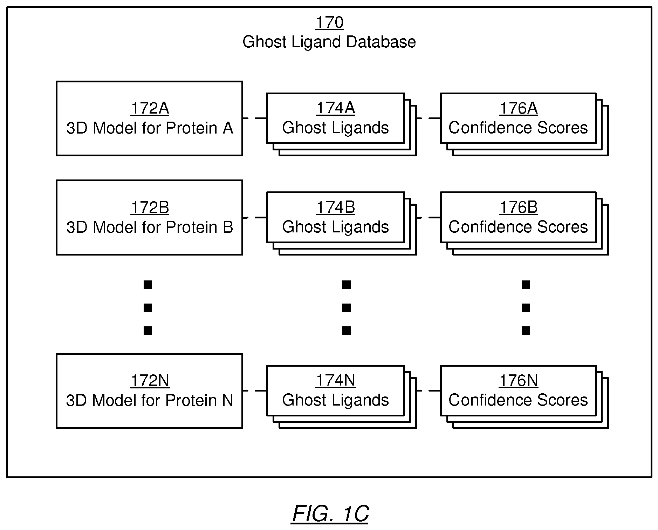

The ghost ligand database (170), in accordance with one or more embodiments, may be any type of storage unit and/or device (*e.g.,* a file system, database, collection of tables, or any other storage mechanism) for storing data. The ghost ligand database (170) is described below with reference to FIG. 1C.

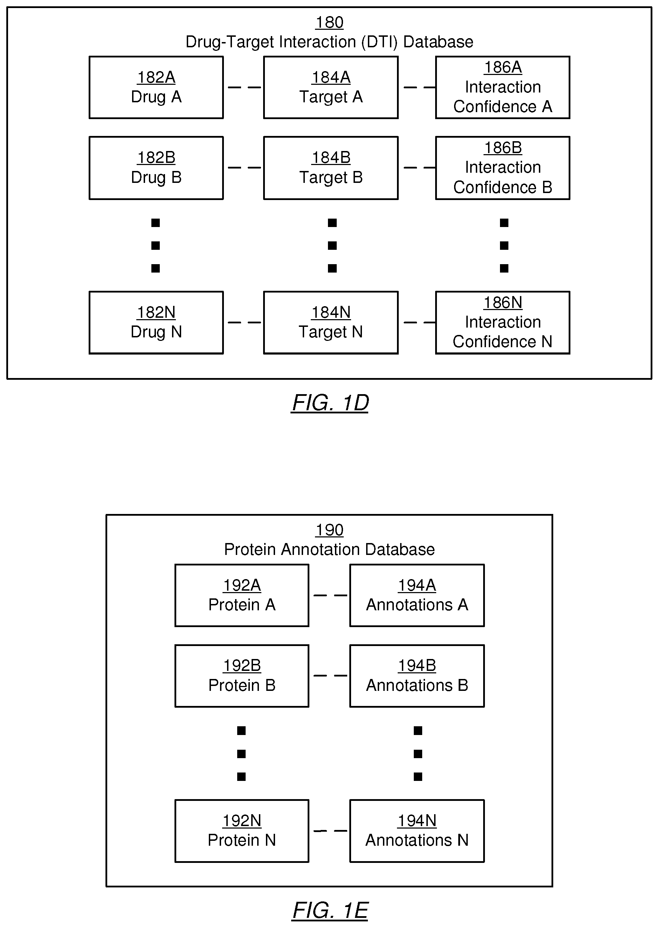

The drug-target interaction database (180), in accordance with one or more embodiments, may be any type of storage unit and/or device (*e.g.,* a file system, database, collection of tables, or any other storage mechanism) for storing data. The drug-target interaction database (180) is described below with reference to FIG. 1D.

The protein annotation database (190), in accordance with one or more embodiments, may be any type of storage unit and/or device (*e.g.,* a file system, database, collection of tables, or any other storage mechanism) for storing data. The protein annotation database (190) is described below with reference to FIG. 1D.

Turning to FIG. 1B, the protein structure database (160), in accordance with one or more embodiments, is shown. The protein structure database (160) may store 3D models of proteins (162A, 162B, 162N). Each of the 3D models may be associated with homology models (164A, 164B, 164N) and/or experimental models (166A, 166B, 166N). Examples of publicly-available protein structure databases (160) that may be used include, but are not limited to the Protein Data Bank (PDB) and SWISS-MODEL.

Turning to FIG. 1C, the ghost ligand database (170), in accordance with one or more embodiments, is shown. The ghost ligand database (170) may store, for multiple 3D models of proteins (*e.g*., 172A, 172B, 172N), the identified ghost ligands (*e.g*., 174A, 174B, 174N). Further, for each identified ghost ligand, a confidence score (*e.g.,* 176A, 176B, 176N) that may be similarity-based is included. The ghost ligand database may be established as described in FIG. 3.

Turning to FIG. 1D, the drug-target interaction database (180), in accordance with one or more embodiments, is shown. The drug-target interaction database (180) may store, for multiple pairs of a drug (*e.g.,* 182A, 182B, 182N) and a target (*e.g.*, 184A, 184B, 184N), an interaction confidence (*e.g.,* 186A, 186B, 186N). Examples of publicly-available drug-target interaction databases (180) that may be used include, but are not limited to STITCH and ChEMBL. These databases may include many data points (~15,000,000 ChEMBL).

Turning to FIG. 1E, the protein annotation database (190), in accordance with one or more embodiments, is shown. The protein annotation database (190) may store, for multiple proteins (*e.g*., 192A, 192B, 192N), associated annotations (*e.g*., 194A, 192B, 194N). Annotations associated with a protein may include any available information about the protein, and may have been manually or computationally added to the protein annotation database. The UniProt database may be used, for example.

FIG. 2, FIG. 3, FIG. 4, FIG. 5, and FIG. 6 show flowcharts in accordance with one or more embodiments. The flowcharts of FIG. 2, FIG. 3, FIG. 4, and FIG. 5 depict methods for training a machine learning model to predict drug-target interactions, and the flowchart of FIG. 6 depicts a method for using the machine learning model to predict drug-target interactions. One or more of the steps in FIG. 2, FIG. 3, FIG. 4, FIG. 5, and FIG. 6 may be performed by the components of the system (100), discussed above in reference to FIG. 1A. In one or more embodiments, one or more of the steps shown in FIG. 2, FIG. 3, FIG. 4, FIG. 5, and FIG. 6 may be omitted, repeated, and/or performed in a different order than the order shown in FIG. 2, FIG. 3, FIG. 4, FIG. 5, and FIG. 6. Additional steps may further be performed. Accordingly, the scope of the invention should not be considered limited to the specific arrangement of steps shown in FIG. 2, FIG. 3, FIG. 4, FIG. 5, and FIG. 6.

Turning to the flowchart of FIG. 2, a method for generating a machine learning model for predicting drug-target interactions (DTIs) is shown. While FIG. 2 is intended to introduce the major steps toward generating the machine learning model, the subsequently discussed flowcharts provide a more detailed description. After completion of the method of FIG. 2, the resulting machine learning model may be used to make predictions, as described in FIG. 6.

In Step 200, a ghost ligand database is generated from proteins obtained from a protein structure database. A detailed description of Step 200 is provided in FIG. 3.

In Step 202, drug-target interaction (DTI) features are generated, including ligand features and protein features. A detailed description of Step 202 is provided in FIG. 4.

In Step 204, the machine learning model for DTI prediction is generated. A detailed description of Step 204 is provided in FIG. 5.

Turning to the flowchart of FIG. 3, a method for generating a ghost ligand database is described. The subsequently described steps are used to exploit homology relationships between proteins to map known ligands onto the structures of their homologs (both experimental and homology models), termed 'ghosts'.

In Step 300, proteins are obtained from a protein structure database. For each of the proteins, a 3D model may be retrieved.

In Step 302, the obtained proteins are clustered by sequence or domain. In one or more embodiments, protein clusters are defined as any set of two or more proteins sharing similarity in primary sequence or three-dimensional topology, commonly known as a fold. Protein clusterings may be obtained directly from publicly-available databases such as the PDB, SCOP, CATH, PFAM, or Uniprot for example. Protein clusters may be manually created on the basis of sequence similarity using protein sequence alignment tools and clustering tools such as BLAST, CD-HIT, or UCLUST for example. Alternatively, proteins with three-dimensional structure models may be clustered by grouping unrelated proteins that share a common topology or fold.

In Step 304, one of the clusters is selected for further processing.

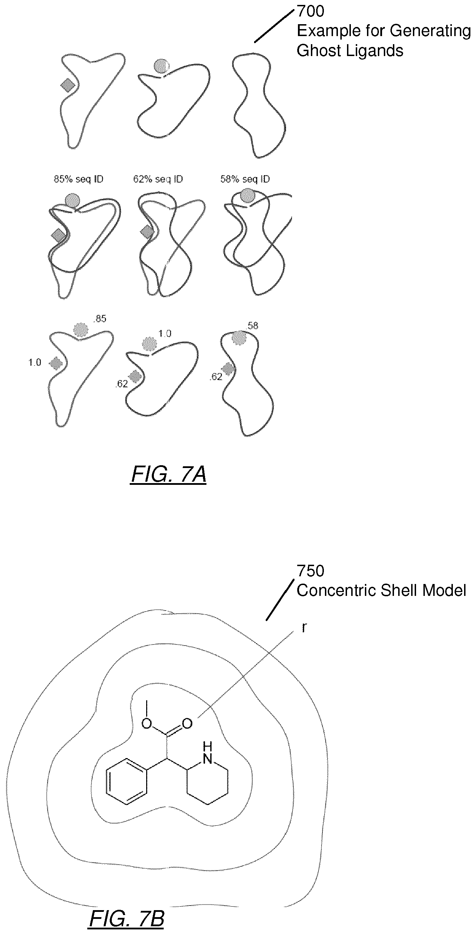

In Step 306, a pairwise structural alignment is performed for all proteins in the selected cluster. FIG. 7A shows examples for the structural alignment of 3D structures of proteins. Three-dimensional (3d) structure alignments attempt to establish positional equivalency between two proteins. Structure alignments may be performed by applying rotational and/or translation transformations to the coordinates of one protein, in order to minimize the average distance between equivalent residues. Structure alignments may be performed on a complete protein structure or a subselection of residues, such as a single domain, or residues surrounding a ligand binding site, for example. The selection of ligand binding site residues for 3d structural alignments is an optimal heuristic approach for mapping ghost ligands.

In Step 308, ghost ligands are obtained by projecting each ligand to the cluster peers. A confidence score may be obtained for each of the projections, comprised of individual scores representing confidence in different components of the ligand projection. Examples for projecting ligands to cluster peers are provided in FIG. 7A. A confidence score may be based on any metrics that quantify uncertainty in the heuristically-defined structural representation selected to model a DTI interaction. At this step, confidence scores may include homology model quality metrics such as percentage sequence identity, sequence similarity, or QMEAN, for example. Confidence scores may also include metrics describing the quality of the structural alignment such as Root Mean Square Deviation for local or global alignment, for example. Multiple confidence scores may also be used.

In Step 310, the ghost ligands and associated confidence scores are stored in the ghost ligand database.

In Step 312, a determination is made about whether additional clusters are remaining for processing. If additional clusters are remaining, the execution of the method may return to Step 304 to select another cluster for processing as described in Steps 306-310. If no additional clusters are remaining, the execution of the method may terminate. Once the method of FIG. 3 terminates, the ghost ligand database may contain a comprehensive collection of the ghost ligands and associated confidence scores for all proteins that were processed as described.

Turning to the flowchart of FIG. 4, a method for generating drug-target interaction (DTI) features is described. The generated features include features for ligands and features for proteins. These features may subsequently be used for training a machine learning model for predicting drug-target interaction. Features for many combinations of proteins and ligands may be generated to ensure the availability of sufficient training samples.

In Step 400, a drug-target interaction for a combination of a ligand and a protein is selected from the DTI database. The subsequent steps are performed for this considered combination of the ligand and the protein. The steps may later be repeated for other combinations of a ligand and a protein.

In Step 402, features are generated for the selected ligand. These features may include a ligand fingerprint and ligand descriptors. The fingerprint may capture the structure of the ligand in a descriptor format and may be based on a SMILES representation of the underlying molecules using a fixed length vector. Molecular fingerprinting methods may include atom pair, extended connectivity fingerprint, graph-based fingerprints, torsion fingerprints, or pharmacophore fingerprints, for example. Molecular weight, number of rotatable bonds, number of hydrogen bond donors, number of hydrogen bond receptors, hydrophobicity, aromaticity, and functional group compositions may be used as ligand descriptors, for example. Molecular shape descriptors such as ellipticity, geometric descriptors, branching descriptors, or chirality descriptors, for example, may also be used.

In Step 404, ghost ligands are retrieved from the ghost ligand database for the selected protein.

In Step 406, each of the ghost ligands is scored, based on its difference to the drug, or more specifically, to the selected ligand. A higher similarity results in a higher score. Similarity between ligands may be scored using a distance metric for comparing molecular fingerprints, such as a Tanimoto distance, D ice distance, or cosine distance for example.

Steps 404 and 406 may be performed for all protein models (*e.g*., homology models or experimental derived models) that are available for the selected protein.

In Step 408, the ghost ligand that is most similar to the selected ligand is selected for further processing.

In Step 410, a confidence vector is generated for the DTI features comprised of individual scores representing confidence in different components of the DTI features and its representative ghost ligand. The confidence vector may include confidence scores representing ghost ligand projections from Step 308. The confidence vector may further include a confidence score for the fingerprint similarity between the selected ligand and the most similar ghost ligand selected in Step 408. In addition, the confidence vector may include a score for the confidence of the selected DTI. The confidence of the selected DTI may be scored based on the source of the DTI data *(e.g.,* a different score may be assigned depending on whether the DTI data were obtained using high or low throughput screening, etc.).

In Step 412, local features are obtained for the selected protein surrounding the most similar ghost ligand. The local features may include binding site features existing in concentric shells of increasing radii, as illustrated in FIG. 7B. For each of the concentric shells, a number of descriptors, *e.g*., atom type descriptors specifying the presence of an atom within the shell are provided. For example, 70 atom type descriptors may be provided per shell radius. The descriptors may further include, but are not limited to a flexibility or rigidity of the binding site within the shell region, residue contacts within the shell region, and/or any other factors that represent the biophysics and indirectly the geometry of the binding site. The features may, however, not specify an exact location or coordinates of atoms. Local features may also include graph descriptions of the ligand binding site, which describe distances between amino acids surrounding the ligand binding site in a network format. Local features may also be defined by the shape of the pocket, corresponding to the void unoccupied by protein residues. The pocket void may be determined by a pocket detection method, such as flood filling, concavity, or solvent accessibility, for example. Local features defined by the pocked void space may include shape of the void space, including volume, ellipticity, curvature, branching patterns, or stability of the space on the basis of the dynamic of nearby residues, for example. Local features defined by residues adjacent to the pocket void space may include orientation of the residues, geometric availability of hydrogen bond donor and acceptor groups, hydrophobicity, aromaticity, or geometric availability of pi stacking interactions, for example. Local features may also include descriptions of the ligand binding channel, which include solvent exposed residues near the ligand binding site that are not in immediate contact with the ligand in the stable bound state. The ligand binding channel is expected to form transient interactions with the ligand in the dynamic process of binding and dissociation. Ligand binding channel features may include features similar to those defining the pocket, such as orientation of residues, amino acid composition, availability of hydrogen bond donors and acceptors, for example.

In Step 414, global features for the structure and/or sequence of the selected protein are obtained by expanding to outer shells with large radii as described in step 412. Shell radii corresponding to local features may include thresholds of 5Å, 10Å, or 15Å for example. Shell radii corresponding to domain-level descriptions or global protein descriptions may have larger distance thresholds of 20Å, 25Å, or 30Å for example, or may have no distance threshold. Global features may further include descriptions of domains or folds and may be derived from publicly-available databases such as SCOP, CATH, or PFAM, for example. Global features may further include features derived from protein sequence and may include the presence of common sequence motifs, for example. Global features may include descriptions of the protein folding state, such as the presence and biophysical properties of intrinsically disordered regions, hinges, loops, ordered regions, or modulatory domains, for example. Global features may also be described with relation to distance from a ligand binding site.

In Step 416, functional annotations of the selected protein are obtained. The functional annotations may be obtained from a protein annotation database. Functional annotations may include Enzyme Commission (EC) numbers, Gene Ontology (GO) annotations, or Uniprot Keywords, for example. Functional annotations may also include the presence or absence of documented positional-specific properties of a protein, such as catalytic sites, post translational modifications, disease associations, or genetic variations, for example.

In Step 418, features are generated for the selected protein. These features may include the local features, the global features and/or the functional annotations.

In Step 420, a determination is made about whether additional DTIs are remaining for processing. If additional DTIs are remaining, the execution of the method may return to Step 400 to select another DTI for processing as described in Steps 402-418. If no additional clusters are remaining, the execution of the method may terminate. Once the method of Fig. 4 terminates, a comprehensive collection of features for the ligands and proteins enumerated in the DTI database is available.

Turning to the flowchart of FIG. 5, a method for generating a machine learning model for DTI prediction is described. Based on the DTI features obtained as described in FIG. 4, a machine learning model that reflects compatibility between a protein environment and ligand attributes is obtained.

In Step 500, ligand features and protein features are obtained. The obtaining of ligand and protein features may be performed as described in Steps 402 and 418 of FIG. 4.

In Step 502, the ligands and proteins are filtered by a function of the confidence vectors established in Step 408 of FIG. 4. The filtering may implement a confidence threshold, with only samples above the confidence threshold being considered for further processing. A confidence function may transform confidence vectors into a single score for filtering. A confidence function may transform confidence scores to probabilities and apply Bayesian statistics to evaluate combined probabilities, for example. A confidence function may apply individual cutoff thresholds to each element of the confidence vector as a means to select which samples are suitable for use in machine learning. Confidence function thresholds or equations may be set by automatically testing different combinations as hyper-parameters of a machine learning algorithm.

In Step 504, ligand and protein features are concatenated to generate positive training samples.

In Step 506, ligand and protein features are shuffled. The shuffled ligand and protein features are concatenated to generate negative training samples. This step may be repeated multiple times to evaluate different ratios of positive-to-negative training samples, 1:1, 1:5, 1:10, 1:19, or 1:20, for example

In Step 508, the machine learning model for DTI prediction is trained using the positive and negative training samples. A learning algorithm based on backpropagation may be used, for example. In one or more embodiments, training samples may be weighted, based on their associated confidence vectors. In one or more embodiments, transfer learning is used to more effectively train the machine learning model. Initially, the machine learning model may be trained by applying an initial confidence threshold in step 502. In subsequent re-training phases, a confidence threshold may be increased to reduce the number and increase the quality of training examples. In addition or alternatively, subsequent re-training phases may limit training examples to select classes of drugs or targets. The machine learning model may be a supervised discriminative classification or regression model such as a random forest, support vector machine, single layer perceptron, or multiple layer artificial neural network, for example. Artificial neural networks are especially well-suited for this task, given the magnitude of training data points (100,000s to 10,000,000s) and the dimensionality of training data features (1000s to 10,000s). In one embodiment, an artificial neural network representation takes the form of fully-connected network with a feature input layer, two hidden layers with, for example, 512 and 256 nodes, respectively, and two output nodes corresponding to interacting and non-interacting pairs. In one embodiment, an artificial neural network with multiple hidden layers omits connections between input types, for the creation of separate latent spaces representing ligand fingerprints, global protein features, local protein features, and protein functional features.

Turning to the flowchart of FIG. 6, a method for predicting an interaction between a query protein and a query ligand is described. The machine learning model trained as described with reference to FIGs. 2-5, may be used to test the "compatibility" of a query protein and a query compound by applying the machine learning model to sets of DTI features corresponding to the query ligand and at least one known binding site of the query protein.

In Step 600, the query protein and the query ligand are obtained. The query protein and the query ligand may be obtained from a user wanting to obtain a prediction of the interaction between the query protein and the query ligand.

In Step 602, possible binding sites and associated local features are obtained for the query protein, as previously described in Steps 404-412 of FIG. 4. Accordingly, one or more binding sites may be obtained from one or more experimental or homology models. Alternatively, the binding sites and associated local features may be obtained from the user, *e.g.,* if the user desires to specify a particular binding site.

In Step 604, global features and protein annotations are obtained for the query protein. The global features and protein annotations may be obtained as previously described in Steps 414 and 416 of FIG. 4.

In Step 606, features are generated for the query protein. These features may include the local features, the global features and/or the functional annotations.

In Step 608, the ligand fingerprint and ligand descriptors are obtained, as previously described in Step 402 of FIG. 4.

In Step 610, features are generated for the query ligand. These features may include the ligand fingerprint and the ligand descriptors.

In Step 612, the machine learning model for DTI prediction is applied to the features of the query ligand and the features of the query protein to obtain a numerical score for a likelihood of interaction between the query ligand and the query protein.

The following paragraphs further illustrate embodiments of the disclosure based on various examples. Those skilled in the art will appreciate that the disclosure is not limited to these examples.

### (i) Sample Ghost Ligands:

Turning to FIG. 7A, an example (700) for generating ghost ligands is shown. Three hypothetical protein structures are shown (top row). Two of the three hypothetical protein structures are in an actual interaction with a ligand (left and center column, top row). The center row shows various structural alignments of the three hypothetical proteins. As a result of the structural alignments, the ligands may be projected to the other proteins. Based on the similarity of the binding site, a confidence score is assigned. The confidence score is 1.0 for the actual ligand-protein pairing, but lower for the ghost ligand-protein pairing. The bottom row shows the resulting ghost ligand-protein pairings as they may be stored in the ghost ligand database.

FIG. 7B shows an illustration of a concentric shell model (750) used to obtain binding site features, in accordance with one or more embodiments. Concentric shells of increasing radii (r) enclose a central chemical structure that is considered part of a binding site. While inner shells capture mostly local features in proximity to the binding site, the outer shells capture increasingly global features. The features representing a protein may be based on the concentric shell model, thus capturing local and global features of the protein, without specifying precise 3D geometries (e.g., on an atomic level).

### (ii) Sample Confidence Vector:

Embodiments of the disclosure rely on a heuristic process to augment drug-target interaction (DTI) data with assumed three-dimensional structure representations. These assumed DTI representations may provide information-rich features for machine learning, improving models aimed at predicting protein-ligand interactions. Obtaining these approximated DTI representations for any given DTI data point requires several assumptions outlined throughout Steps 200 and 202 of FIG. 2. For example, the three-dimensional protein structures used to represent DTIs may be sourced from a homology model rather than directly from experimental coordinates.

Confidence Vectors are comprised of multiple metrics that describe measurable uncertainty in the approximated DTI representations. These metrics are accrued in the creation of the ghost ligand database (Step 200) and projecting known DTI data onto the ghost ligand database (Step 202). In one example, the Confidence Vector contains four elements, including: (1) Percentage of sequence identity between the homology model representing the DTI protein and its source template, (2) RMSD from the alignment between the source structure of the ghost ligand and the homology model representing the DTI protein, (3) Tanimoto similarity between morgan3 fingerprints of the DTI ligand and the ghost ligand template (4) confidence in the DTI data point.

In the example, a Drug-Target Interaction (DTI) database indicates that ligand gefitinib, interacts with protein Aurora Kinase A. The DTI database assigned the interaction a probability of 85%, based accuracy of the source biophysical experiment. The specific interaction between gefitinib and Aurora Kinase A is not present in the source three-dimensional structure database. In creating the ghost ligand database, a homology model for the Aurora Kinase A protein was created from close homolog Aurora Kinase B, which shares a sequence identity of 72.5%. The closest molecule to gefitinib, that was successfully mapped onto the homology model was erlotinib which shares a Morgan3 fingerprint Tanimoto similarity of 0.372. The erlotinib ghost ligand location was approximated based on a structural alignment between the Aurora Kinase A homology model and erlotinib-EGFR co-complex crystal structure, with a ligand binding site RMSD of 2.345Å. Accordingly, the corresponding confidence vector would be: [85%, 72.5%, 0.372, 2.345Å].

### (iii) Sample Training Data and Negative Shuffling:

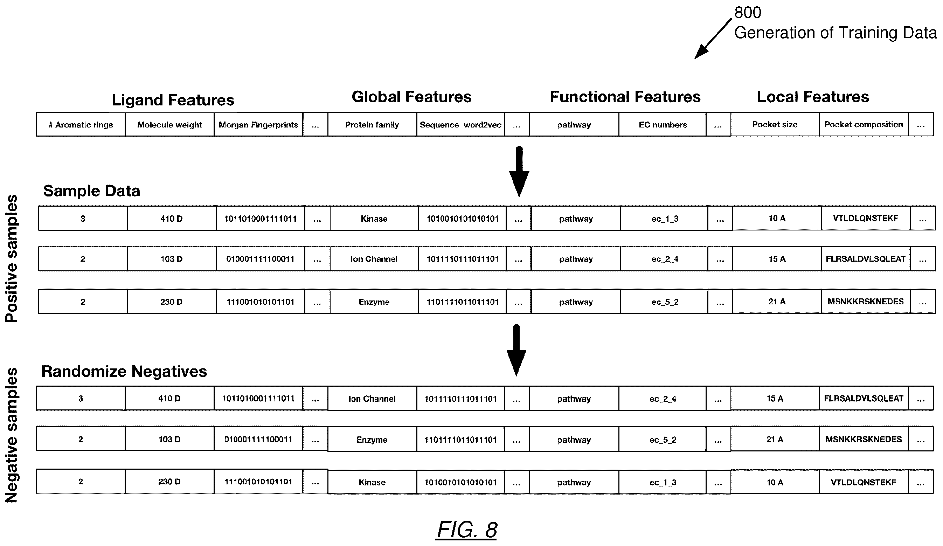

The described methodology focuses on augmenting drug-target interacting (DTI) pairs from a DTI database with a mixture of relevant features obtained through a series of deterministic mappable relationships and heuristically modeled features (local structure features). Each row in a DTI database may be transformed into a feature vector as exemplified in FIG. 8., showing the generation of training data (800), with ligand features from the corresponding drug (columns labeled "Ligand Features") and protein features (columns labeled "Global Features", "Functional Features, and "Local Features"). Global and functional features of the protein may be retrieved from any protein using standard practices of database lookups and protein identifier mapping. The local protein features may be the outcome of the heuristically-defined process outlined in this patent. They are modeled and therefore may be inaccurate. Each data row also has a corresponding confidence vector (described above but not shown in figure), which may be used to imply hard cutoffs or weights for training a machine learning model.

When a neural network is trained only by true drug-target interactions positive examples, extracted from drug-target interaction datasets, the model might learn to ignore the core and obvious patterns of interactions since they will not provide any signal to the model. Further, it may be necessary to control a potentially significant bias toward highly represented drugs and targets in drug-target interaction datasets. Hence, it may be beneficial to sample negative examples with the relative proportion for each drug and target. As a result, the model may learn patterns based on positive examples and negative examples. In FIG. 8, the randomized negatives shuffle the ligand components of the feature vector (white) with the protein component of the feature vector (three shades of gray). The resulting negative examples may be used for training a classification engine, balancing the presence of individual ligand or protein features. The equal use of individual ligand and/or protein features in the positive and negative sets avoids the network learning that any individual feature is particularly associated with binding in general.

Embodiments of the disclosure use ghost ligands to create local protein features for proteochemometrics (PCM) from protein-ligand datasets. More specifically, drug-target interactions (DTI) data are threaded onto 3D atomistic models of protein-ligand complexes to derive local protein features. Mixed feature datasets of the PCMs may include local (pocket), regional (domain) and global (whole protein) annotations and/or functional annotations.

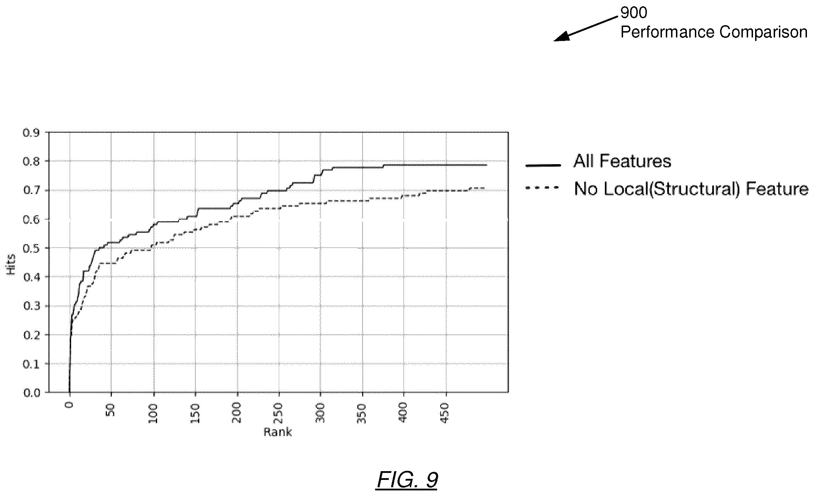

Conventionally, training data for machine learning should be high-confidence 'model quality data'. The use of a prediction (ghost ligands + threading), in accordance with one or more embodiments, to produce training data for a machine learning algorithm appears to be counterintuitive. Specifically, if the heuristic approach is not sufficiently accurate, then, conventional wisdom suggests that the introduction of local features derived from the combination of ghost ligand and threading has the potential to introduce additional noise, thereby reducing the performance over conventional DTI PMCs. However, as the performance comparison (900) of FIG. 9 shows, an increase in performance is achieved by the introduction of local features derived through the approach described in this patent. Omitting local features derived by this approach is equivalent to the performance that would be achieved through DTI PMCs alone.

Specifically, FIG. 9 shows a performance comparison for ranking a likelihood of binding for a small molecule ligand and 8717 proteins. To test ranking, 100 molecules are randomly removed from training data and used for testing. This figure plots the predicted rank of known interactions for these 100 random drugs. Without local features for example, only ~63% of real interactions are observed among predictions for the top 300 proteins of 8717 (top ~3.5%) (dotted line). Including the local features, estimated by this procedure, the discovery rate increased to ~75% for the same threshold (solid line).

Various embodiments of the disclosure have one or more of the following advantages. Embodiments of the invention enable the prediction of drug-target interactions (DTIs) using machine learning models that reflect compatibility between a protein environment and ligand attributes. Localized 3D features are created to represent binding sites, even when there is no 3D information available for the interaction under consideration.

Mapping known drug-target interactions onto homology models synthetically augments abundant DTI training data with highly-dimensional biophysical information to train a deep neural network. The approach, thus, enables the use of comprehensive DTI databases, even though it is not necessarily known where a drug binds to a protein, for the entries of the DTI databases.

Methods in accordance with one or more embodiments do not require in-detail knowledge of the biophysics of protein-ligand interactions. Accordingly, no precise 3D coordinates of atoms are required, thereby enabling the use of DTI databases and homology models to map drug-target interactions onto pockets of proteins.

Embodiments of the disclosure require a reduced feature space and permit vastly larger training data in comparison to structure-based deep learning approaches relying on 3D atomic coordinates. Further, embodiments of the disclosure were found to generalize well. An initial performance evaluation suggests that the method as described performs about 1,000,000 times faster, in comparison to docking simulations. The methods in accordance with one or more embodiment require no human intervention. Specifically, the most likely protein representation and binding site is automatically identified. As discussed in Exhibits A and B, methods in accordance with one or more embodiments may be used as accurate in-silico alternatives or additions to other in-silico and/or experimental methods for predicting drug-target interactions.

Embodiments of the disclosure may have various applications. For example, embodiments may be used for proteome screenings (*e.g*., to perform toxicity predictions or phenotypic deconvolution predictions), virtual screenings, and for drug discovery and development in general.

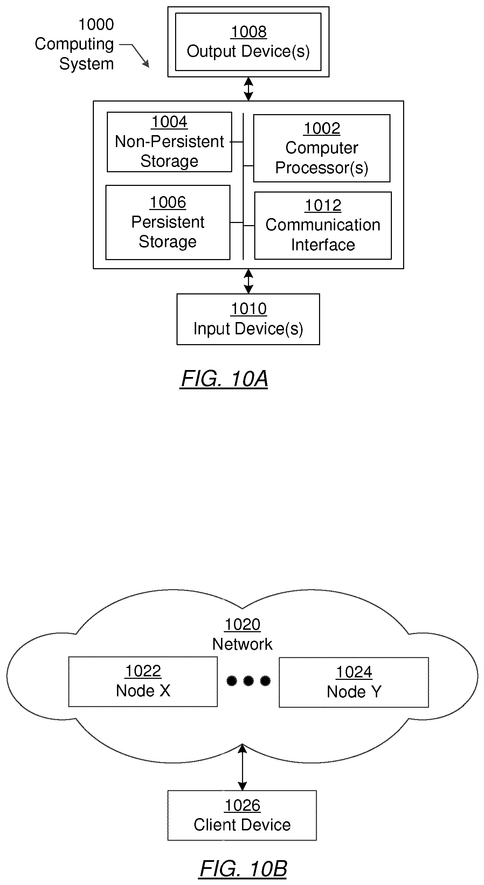

Embodiments of the disclosure may be implemented on a computing system. Any combination of mobile, desktop, server, router, switch, embedded device, or other types of hardware may be used. For example, as shown in FIG. 10A, the computing system (1000) may include one or more computer processors (1002), non-persistent storage (1004) *(e.g.,* volatile memory, such as random access memory (RAM), cache memory), persistent storage (1006) *(e.g.,* a hard disk, an optical drive such as a compact disk (CD) drive or digital versatile disk (DVD) drive, a flash memory, etc.), a communication interface (1012) *(e.g.,* Bluetooth interface, infrared interface, network interface, optical interface, etc.), and numerous other elements and functionalities.

The computer processor(s) (1002) may be an integrated circuit for processing instructions. For example, the computer processor(s) may be one or more cores or micro-cores of a processor. The computing system (1000) may also include one or more input devices (1010), such as a touchscreen, keyboard, mouse, microphone, touchpad, electronic pen, or any other type of input device.

The communication interface (1012) may include an integrated circuit for connecting the computing system (1000) to a network (not shown) (*e.g*., a local area network (LAN), a wide area network (WAN) such as the Internet, mobile network, or any other type of network) and/or to another device, such as another computing device.

Further, the computing system (1000) may include one or more output devices (1008), such as a screen (*e.g.,* a liquid crystal display (LCD), a plasma display, touchscreen, cathode ray tube (CRT) monitor, projector, or other display device), a printer, external storage, or any other output device. One or more of the output devices may be the same or different from the input device(s). The input and output device(s) may be locally or remotely connected to the computer processor(s) (1002), non-persistent storage (1004), and persistent storage (1006). Many different types of computing systems exist, and the aforementioned input and output device(s) may take other forms.

Software instructions in the form of computer readable program code to perform embodiments of the disclosure may be stored, in whole or in part, temporarily or permanently, on a non-transitory computer readable medium such as a CD, DVD, storage device, a diskette, a tape, flash memory, physical memory, or any other computer readable storage medium. Specifically, the software instructions may correspond to computer readable program code that, when executed by a processor(s), is configured to perform one or more embodiments of the disclosure.

The computing system (1000) in FIG. 10A may be connected to or be a part of a network. For example, as shown in FIG. 10B, the network (1020) may include multiple nodes (*e.g.,* node X (1022), node Y (1024)). Each node may correspond to a computing system, such as the computing system shown in FIG. 10A, or a group of nodes combined may correspond to the computing system shown in FIG. 10A. By way of an example, embodiments of the disclosure may be implemented on a node of a distributed system that is connected to other nodes. By way of another example, embodiments of the disclosure may be implemented on a distributed computing system having multiple nodes, where each portion of the disclosure may be located on a different node within the distributed computing system. Further, one or more elements of the aforementioned computing system (1000) may be located at a remote location and connected to the other elements over a network.

Although not shown in FIG. 10B, the node may correspond to a blade in a server chassis that is connected to other nodes via a backplane. By way of another example, the node may correspond to a server in a data center. By way of another example, the node may correspond to a computer processor or micro-core of a computer processor with shared memory and/or resources.

The nodes *(e.g.,* node X (1022), node Y (1024)) in the network (1020) may be configured to provide services for a client device (1026). For example, the nodes may be part of a cloud computing system. The nodes may include functionality to receive requests from the client device (1026) and transmit responses to the client device (1026). The client device (1026) may be a computing system, such as the computing system shown in FIG. 10A. Further, the client device (1026) may include and/or perform all or a portion of one or more embodiments of the disclosure.

The computing system or group of computing systems described in FIG. 10A and 10B may include functionality to perform a variety of operations disclosed herein. For example, the computing system(s) may perform communication between processes on the same or different system. A variety of mechanisms, employing some form of active or passive communication, may facilitate the exchange of data between processes on the same device. Examples representative of these inter-process communications include, but are not limited to, the implementation of a file, a signal, a socket, a message queue, a pipeline, a semaphore, shared memory, message passing, and a memory-mapped file. Further details pertaining to a couple of these nonlimiting examples are provided below.

Based on the client-server networking model, sockets may serve as interfaces or communication channel end-points enabling bidirectional data transfer between processes on the same device. Foremost, following the client-server networking model, a server process (*e.g.,* a process that provides data) may create a first socket object. Next, the server process binds the first socket object, thereby associating the first socket object with a unique name and/or address. After creating and binding the first socket object, the server process then waits and listens for incoming connection requests from one or more client processes (*e.g*., processes that seek data). At this point, when a client process wishes to obtain data from a server process, the client process starts by creating a second socket object. The client process then proceeds to generate a connection request that includes at least the second socket object and the unique name and/or address associated with the first socket object. The client process then transmits the connection request to the server process. Depending on availability, the server process may accept the connection request, establishing a communication channel with the client process, or the server process, busy in handling other operations, may queue the connection request in a buffer until server process is ready. An established connection informs the client process that communications may commence. In response, the client process may generate a data request specifying the data that the client process wishes to obtain. The data request is subsequently transmitted to the server process. Upon receiving the data request, the server process analyzes the request and gathers the requested data. Finally, the server process then generates a reply including at least the requested data and transmits the reply to the client process. The data may be transferred, more commonly, as datagrams or a stream of characters (*e.g.*, bytes).

Shared memory refers to the allocation of virtual memory space in order to substantiate a mechanism for which data may be communicated and/or accessed by multiple processes. In implementing shared memory, an initializing process first creates a shareable segment in persistent or non-persistent storage. Post creation, the initializing process then mounts the shareable segment, subsequently mapping the shareable segment into the address space associated with the initializing process. Following the mounting, the initializing process proceeds to identify and grant access permission to one or more authorized processes that may also write and read data to and from the shareable segment. Changes made to the data in the shareable segment by one process may immediately affect other processes, which are also linked to the shareable segment. Further, when one of the authorized processes accesses the shareable segment, the shareable segment maps to the address space of that authorized process. Often, only one authorized process may mount the shareable segment, other than the initializing process, at any given time.

Other techniques may be used to share data, such as the various data described in the present application, between processes without departing from the scope of the disclosure. The processes may be part of the same or different application and may execute on the same or different computing system.

Rather than or in addition to sharing data between processes, the computing system performing one or more embodiments of the disclosure may include functionality to receive data from a user. For example, in one or more embodiments, a user may submit data via a graphical user interface (GUI) on the user device. Data may be submitted via the graphical user interface by a user selecting one or more graphical user interface widgets or inserting text and other data into graphical user interface widgets using a touchpad, a keyboard, a mouse, or any other input device. In response to selecting a particular item, information regarding the particular item may be obtained from persistent or non-persistent storage by the computer processor. Upon selection of the item by the user, the contents of the obtained data regarding the particular item may be displayed on the user device in response to the user's selection.

By way of another example, a request to obtain data regarding the particular item may be sent to a server operatively connected to the user device through a network. For example, the user may select a uniform resource locator (URL) link within a web client of the user device, thereby initiating a Hypertext Transfer Protocol (HTTP) or other protocol request being sent to the network host associated with the URL. In response to the request, the server may extract the data regarding the particular selected item and send the data to the device that initiated the request. Once the user device has received the data regarding the particular item, the contents of the received data regarding the particular item may be displayed on the user device in response to the user's selection. Further to the above example, the data received from the server after selecting the URL link may provide a web page in Hyper Text Markup Language (HTML) that may be rendered by the web client and displayed on the user device.

Once data is obtained, such as by using techniques described above or from storage, the computing system, in performing one or more embodiments of the disclosure, may extract one or more data items from the obtained data. For example, the extraction may be performed as follows by the computing system in FIG. 10A. First, the organizing pattern (*e.g*., grammar, schema, layout) of the data is determined, which may be based on one or more of the following: position (*e.g*., bit or column position, Nth token in a data stream, etc.), attribute (where the attribute is associated with one or more values), or a hierarchical/tree structure (including layers of nodes at different levels of detail-such as in nested packet headers or nested document sections). Then, the raw, unprocessed stream of data symbols is parsed, in the context of the organizing pattern, into a stream (or layered structure) of tokens (where each token may have an associated token "type").

Next, extraction criteria are used to extract one or more data items from the token stream or structure, where the extraction criteria are processed according to the organizing pattern to extract one or more tokens (or nodes from a layered structure). For position-based data, the token(s) at the position(s) identified by the extraction criteria are extracted. For attribute/value-based data, the token(s) and/or node(s) associated with the attribute(s) satisfying the extraction criteria are extracted. For hierarchical/layered data, the token(s) associated with the node(s) matching the extraction criteria are extracted. The extraction criteria may be as simple as an identifier string or may be a query provided to a structured data repository (where the data repository may be organized according to a database schema or data format, such as XML).

The extracted data may be used for further processing by the computing system. For example, the computing system of FIG. 10A, while performing one or more embodiments of the disclosure, may perform data comparison. Data comparison may be used to compare two or more data values (*e.g.,* A, B). For example, one or more embodiments may determine whether A > B, A = B, A != B, A < B, etc. The comparison may be performed by submitting A, B, and an opcode specifying an operation related to the comparison into an arithmetic logic unit (ALU) (*i.e.,* circuitry that performs arithmetic and/or bitwise logical operations on the two data values). The ALU outputs the numerical result of the operation and/or one or more status flags related to the numerical result. For example, the status flags may indicate whether the numerical result is a positive number, a negative number, zero, etc. By selecting the proper opcode and then reading the numerical results and/or status flags, the comparison may be executed. For example, in order to determine if A > B, B may be subtracted from A (*i.e.,* A - B), and the status flags may be read to determine if the result is positive (*i.e.,* if A > B, then A - B > 0). In one or more embodiments, B may be considered a threshold, and A is deemed to satisfy the threshold if A = B or if A > B, as determined using the ALU. In one or more embodiments of the disclosure, A and B may be vectors, and comparing A with B requires comparing the first element of vector A with the first element of vector B, the second element of vector A with the second element of vector B, etc. In one or more embodiments, if A and B are strings, the binary values of the strings may be compared.

The computing system in FIG. 10A may implement and/or be connected to a data repository. For example, one type of data repository is a database. A database is a collection of information configured for ease of data retrieval, modification, re-organization, and deletion. Database Management System (DBMS) is a software application that provides an interface for users to define, create, query, update, or administer databases.

The user, or software application, may submit a statement or query into the DBMS. Then the DBMS interprets the statement. The statement may be a select statement to request information, update statement, create statement, delete statement, etc. Moreover, the statement may include parameters that specify data, or data container (database, table, record, column, view, etc.), identifier(s), conditions (comparison operators), functions (*e.g.* join, full join, count, average, etc.), sort (*e.g.* ascending, descending), or others. The DBMS may execute the statement. For example, the DBMS may access a memory buffer, a reference or index a file for read, write, deletion, or any combination thereof, for responding to the statement. The DBMS may load the data from persistent or non-persistent storage and perform computations to respond to the query. The DBMS may return the result(s) to the user or software application.

The computing system of FIG. 10A may include functionality to provide raw and/or processed data, such as results of comparisons and other processing. For example, providing data may be accomplished through various presenting methods. Specifically, data may be provided through a user interface provided by a computing device. The user interface may include a GUI that displays information on a display device, such as a computer monitor or a touchscreen on a handheld computer device. The GUI may include various GUI widgets that organize what data is shown as well as how data is provided to a user. Furthermore, the GUI may provide data directly to the user, *e.g.,* data provided as actual data values through text, or rendered by the computing device into a visual representation of the data, such as through visualizing a data model.

For example, a GUI may first obtain a notification from a software application requesting that a particular data object be provided within the GUI. Next, the GUI may determine a data object type associated with the particular data object, *e.g.*, by obtaining data from a data attribute within the data object that identifies the data object type. Then, the GUI may determine any rules designated for displaying that data object type, *e.g.,* rules specified by a software framework for a data object class or according to any local parameters defined by the GUI for presenting that data object type. Finally, the GUI may obtain data values from the particular data object and render a visual representation of the data values within a display device according to the designated rules for that data object type.

Data may also be provided through various audio methods. In particular, data may be rendered into an audio format and provided as sound through one or more speakers operably connected to a computing device.

Data may also be provided to a user through haptic methods. For example, haptic methods may include vibrations or other physical signals generated by the computing system. For example, data may be provided to a user using a vibration generated by a handheld computer device with a predefined duration and intensity of the vibration to communicate the data.

The above description of functions presents only a few examples of functions performed by the computing system of FIG. 10A and the nodes and/ or client device in FIG. 10B. Other functions may be performed using one or more embodiments of the disclosure.

While the disclosure has been described with respect to a limited number of embodiments, those skilled in the art, having benefit of this disclosure, will appreciate that other embodiments can be devised which do not depart from the scope of the disclosure as disclosed herein. Accordingly, the scope of the disclosure should be limited only by the attached claims.

The embodiments and examples set forth herein were presented in order to best explain the present invention and its particular application and to thereby enable those skilled in the art to make and use the invention. However, those skilled in the art will recognize that the foregoing description and examples have been presented for the purposes of illustration and example only. The description as set forth is not intended to be exhaustive or to limit the invention to the precise form disclosed.

While the invention has been described with respect to a limited number of embodiments, those skilled in the art, having benefit of this disclosure, will appreciate that other embodiments can be devised which do not depart from the scope of the invention as disclosed herein. Accordingly, the scope of the invention should be limited only by the attached claims.

## Claims

1. A computer-implemented method for predicting drug-target binding using synthetically-augmented data, the method comprising:
generating a plurality of ghost ligands for a plurality of proteins in a protein structure database,
wherein generating the plurality of ghost ligands comprises:
for a cluster of proteins, selected from the plurality of proteins:
performing a structure alignment for the proteins in the cluster of proteins;
obtaining the plurality of ghost ligands by projecting a ligand of one of the proteins in the cluster onto all other proteins in the cluster, after the structure alignment;
obtaining, for each of the plurality of ghost ligands, a confidence score;
generating a plurality of drug-target interaction (DTI) features for proteins and ligands in a DTI database, using the plurality of ghost ligands;
generating a machine learning model using the plurality of DTI features, wherein generating the machine learning model comprises:
obtaining positive training samples based on the plurality of DTI features for the proteins and the ligands;
obtaining negative training samples based on the plurality of DTI features by shuffling, at least once, the plurality of DTI features for the proteins and the ligands;
using the positive and the negative training samples, training the machine learning model for DTI prediction; and
predicting a likelihood of interaction for a combination of a query protein and a query ligand using the machine learning model.

2. The method of claim 1, wherein the cluster of proteins is obtained based on one selected from the group consisting of:
a similarity in sequence,
a similarity in three-dimensional topology, and
an existing clustering in a database.

3. The method of claim 1, wherein the confidence score quantifies an uncertainty of the associated ghost ligand.

4. The method of claim 1, wherein generating the plurality of DTI features comprises:
for each of a plurality of combinations of a ligand and a protein in the DTI database:
selecting, from the plurality of ghost ligands, a ghost ligand that is most similar to the ligand considered for the combination;
optionally, wherein the selection of the most similar ghost ligand is performed based on a distance metric; and
generating features for the protein considered for the combination, wherein the generated features characterize the protein considered for the combination.

5. The method of claim 4, wherein the generated features comprise one selected from the group consisting of:
at least one local feature comprising binding site features in concentric shells of increasing radii,
at least one global feature beyond the binding site features, and
at least one functional annotation.

6. The method of claim 4, wherein generating the plurality of DTI features further comprises:
obtaining a confidence vector representing a confidence in a plurality of components of the DTI features associated with the most similar ghost ligand;
optionally, wherein the confidence in the plurality of components comprises at least one selected from the group consisting of:
a first confidence score quantifying an uncertainty associated with the most similar ghost ligand,
a second confidence score quantifying a fingerprint similarity between the ligand considered for the combination and the most similar ghost ligand, and
a third confidence score depending on the source from which the DTI features are obtained.

7. The method of claim 1, wherein generating the machine learning model comprises,
prior to obtaining the positive training samples and the negative training samples:
filtering the plurality of DTI features for the proteins and the ligands using a confidence threshold applied to confidence vectors associated with the plurality of DTI features.

8. The method of claim 1, wherein the machine learning model is one selected from the group consisting of a classifier model and a regression model.

9. The method of claim 1, wherein predicting the likelihood of interaction for the combination of the query protein and the query ligand comprises:
obtaining, for the query protein, a possible binding site and associated local features based on the plurality of ghost ligands;
generating features for the query protein, the features for the query protein comprising the local features;
generating features for the query ligand, the features for the query ligand comprising a ligand fingerprint and a ligand descriptor; and
applying the machine learning model to the features of the query protein and the features of the query ligand to obtain a likelihood of interaction between the query ligand and the query protein;
optionally, wherein the features for the query protein further comprise at least one selected from the group consisting of global features and functional annotations.

10. A non-transitory computer readable medium comprising computer readable program code for predicting drug-target binding using synthetically-augmented data, the computer readable program code causing a computer system to:
generate a plurality of ghost ligands for a plurality of proteins in a protein structure database,
wherein generating the plurality of ghost ligands comprises:
for a cluster of proteins, selected from the plurality of proteins:
performing a structure alignment for the proteins in the cluster of proteins;
obtaining the plurality of ghost ligands by projecting a ligand of one of the proteins in the cluster onto all other proteins in the cluster, after the structure alignment;
obtaining, for each of the plurality of ghost ligands, a confidence score;
generate a plurality of drug-target interaction (DTI) features for proteins and ligands in a DTI database, using the plurality of ghost ligands;
generate a machine learning model using the DTI features, wherein generating the machine learning model comprises:
obtaining positive training samples based on the plurality of DTI features for the proteins and the ligands;
obtaining negative training samples based on the plurality of DTI features by shuffling, at least once, the plurality of DTI features for the proteins and the ligands;
using the positive and the negative training samples, training the machine learning model for DTI prediction; and
predict a likelihood of interaction for a combination of a query protein and a query ligand using the machine learning model.

11. A system for differential drug discovery, the system comprising:
a protein structure database;
a ghost ligand identification engine configured to generate a plurality of ghost ligands for a plurality of proteins in the protein structure database,
wherein generating the plurality of ghost ligands comprises:
for a cluster of proteins, selected from the plurality of proteins:
performing a structure alignment for the proteins in the cluster of proteins;
obtaining the plurality of ghost ligands by projecting a ligand of one of the proteins in the cluster onto all other proteins in the cluster, after the structure alignment;
obtaining, for each of the plurality of ghost ligands, a confidence score;
a ghost ligand database storing the plurality of ghost ligands;
a drug-target interaction (DTI) database storing proteins and ligand;
a feature generation engine configured to generate a plurality of DTI features for the proteins and the ligands in the DTI database, using the plurality of ghost ligands in the ghost ligand database;
a machine learning model training engine configured to generate a machine learning model using the DTI features, wherein generating the machine learning model comprises:
obtaining positive training samples based on the plurality of DTI features for the proteins and the ligands;
obtaining negative training samples based on the plurality of DTI features by shuffling, at least once, the plurality of DTI features for the proteins and the ligands;
using the positive and the negative training samples, training the machine learning model for DTI prediction; and
a DTI prediction engine configured to predict a likelihood of interaction for a combination of a query protein and a query ligand using the machine learning model.

12. The system of claim 11, wherein generating the plurality of DTI features comprises:
for each of a plurality of combinations of a ligand and a protein in the DTI database:
selecting, from the plurality of ghost ligands, a ghost ligand that is most similar to the ligand considered for the combination; and
generating features for the protein considered for the combination, wherein the generated features characterize the protein considered for the combination.

13. The system of claim 12, wherein the generated features comprise one selected from the group consisting of:
at least one local feature comprising binding site features in concentric shells of increasing radii,
at least one global feature beyond the binding site features, and
at least one functional annotation;
optionally, wherein generating the plurality of DTI features further comprises:
obtaining a confidence vector representing a confidence in a plurality of components of the DTI features associated with the most similar ghost ligand,
wherein the confidence in the plurality of components comprises at least one selected from the group consisting of:
a first confidence score quantifying an uncertainty associated with the most similar ghost ligand,
a second confidence score quantifying a fingerprint similarity between the ligand considered for the combination and the most similar ghost ligand, and
a third confidence score depending on the source from which the DTI features are obtained.

14. The system of claim 11, wherein predicting the likelihood of interaction for the combination of the query protein and the query ligand comprises:
obtaining, for the query protein, a possible binding site and associated local features based on the plurality of ghost ligands;
generating features for the query protein, the features for the query protein comprising the local features;
generating features for the query ligand, the features for the query ligand comprising a ligand fingerprint and a ligand descriptor; and
applying the machine learning model to the features of the query protein and the features of the query ligand to obtain a likelihood of interaction between the query ligand and the query protein.

## Patentansprüche

1. Computerimplementiertes Verfahren zum Vorhersagen einer Wirkstoff-Ziel-Bindung unter Verwendung synthetisch erweiterter Daten, wobei das Verfahren umfasst:
Erzeugen einer Mehrzahl von Ghost-Liganden für eine Mehrzahl von Proteinen in einer Proteinstrukturdatenbank,
wobei das Erzeugen der Mehrzahl von Ghost-Liganden umfasst:
für einen Cluster von Proteinen, der aus der Mehrzahl von Proteinen ausgewählt ist: Durchführen eines Strukturalignments für die Proteine in dem Proteincluster;
Erhalten der Mehrzahl von Ghost-Liganden durch Projizieren eines Liganden von einem der Proteine in dem Cluster auf alle anderen Proteine in dem Cluster nach dem Strukturalignment;
Erhalten, für jeden der Mehrzahl von Ghost-Liganden, eines Konfidenzwerts;
Erzeugen einer Mehrzahl von Merkmalen einer Wirkstoff-Ziel-Interaktion (DTI) für Proteine und Liganden in einer DTI-Datenbank unter Verwendung der Mehrzahl von Ghost-Liganden;
Erzeugen eines Modells für maschinelles Lernen unter Verwendung der Mehrzahl von DTI-Merkmalen, wobei das Erzeugen des Modells für maschinelles Lernen umfasst:
Erhalten positiver Trainingsbeispiele auf der Grundlage der Mehrzahl von DTI-Merkmalen für die Proteine und die Liganden;
Erhalten negativer Trainingsbeispiele auf der Grundlage der Mehrzahl von DTI-Merkmalen durch mindestens einmaliges Durchmischen der Mehrzahl von DTI-Merkmalen für die Proteine und die Liganden;
unter Verwendung der positiven und der negativen Trainingsbeispiele, Trainieren des Modells für maschinelles Lernen für die DTI-Vorhersage; und
Vorhersagen einer Wahrscheinlichkeit einer Interaktion für eine Kombination aus einem Abfrageprotein und einem Abfrageliganden unter Verwendung des Modells für maschinelles Lernen.

2. Verfahren nach Anspruch 1, wobei der Proteincluster auf der Grundlage von einer erhalten wird, die ausgewählt ist aus der Gruppe bestehend aus:
einer Sequenzähnlichkeit,
einer Ähnlichkeit der dreidimensionalen Topologie, und
einer vorhandenen Clusterbildung in einer Datenbank.

3. Verfahren nach Anspruch 1, wobei der Konfidenzwert eine Unsicherheit des zugehörigen Ghost-Liganden quantifiziert.

4. Verfahren nach Anspruch 1, wobei das Erzeugen der Mehrzahl von DTI-Merkmalen umfasst:
für jede einer Mehrzahl von Kombinationen aus einem Liganden und einem Protein in der DTI-Datenbank:
Auswählen, aus der Mehrzahl von Ghost-Liganden, eines Ghost-Liganden, der dem für die Kombination in Betracht gezogenen Liganden am ähnlichsten ist;
wobei optional die Auswahl des ähnlichsten Ghost-Liganden auf der Grundlage einer Abstandsmetrik durchgeführt wird; und
Erzeugen von Merkmalen für das für die Kombination in Betracht gezogene Protein, wobei die erzeugten Merkmale das für die Kombination in Betracht gezogene Protein charakterisieren.

5. Verfahren nach Anspruch 4, wobei die erzeugten Merkmale eines umfassen, das ausgewählt ist aus der Gruppe bestehend aus:
mindestens einem lokalen Merkmal, das Bindungsstellenmerkmale in konzentrischen Schichten mit zunehmendem Radius umfasst,
mindestens einem globalen Merkmal zusätzlich zu den Bindungsstellenmerkmalen, und
mindestens einer funktionellen Annotation.

6. Verfahren nach Anspruch 4, wobei das Erzeugen der Mehrzahl von DTI-Merkmalen weiter umfasst:
Erhalten eines Konfidenzvektors, der eine Konfidenz in einer Mehrzahl von Komponenten der DTI-Merkmale darstellt, die dem ähnlichsten Ghost-Liganden zugeordnet sind;
wobei optional die Konfidenz in der Mehrzahl von Komponenten mindestens einen umfasst, der ausgewählt ist aus der Gruppe bestehend aus:
einem ersten Konfidenzwert, der eine Unsicherheit quantifiziert, die dem ähnlichsten Ghost-Liganden zugeordnet ist,
einem zweiten Konfidenzwert, der eine Ähnlichkeit der Fingerabdrücke zwischen dem für die Kombination in Betracht gezogenen Liganden und dem ähnlichsten Ghost-Liganden quantifiziert, und
einem dritten Konfidenzwert, der von der Quelle abhängig ist, aus der die DTI-Merkmale erhalten werden.

7. Verfahren nach Anspruch 1, wobei das Erzeugen des Modells für maschinelles Lernen umfasst,
vor dem Erhalten der positiven Trainingsbeispiele und der negativen Trainingsbeispiele:
Filtern der Mehrzahl von DTI-Merkmalen für die Proteine und die Liganden unter Verwendung eines Konfidenzschwellenwerts, der auf zu der Mehrzahl von DTI-Merkmalen zugehörige Konfidenzvektoren angewendet wird.

8. Verfahren nach Anspruch 1, wobei das Modell für maschinelles Lernen ausgewählt ist aus der Gruppe bestehend aus einem Klassifikationsmodell und einem Regressionsmodell.

9. Verfahren nach Anspruch 1, wobei das Vorhersagen der Wahrscheinlichkeit einer Interaktion für die Kombination aus dem Abfrageprotein und dem Abfrageliganden umfasst:
Erhalten, für das Abfrageprotein, einer möglichen Bindungsstelle und zugehöriger lokaler Merkmale auf der Grundlage der Mehrzahl von Ghost-Liganden;
Erzeugen von Merkmalen für das Abfrageprotein, wobei die Merkmale für das Abfrageprotein die lokalen Merkmale umfassen;
Erzeugen von Merkmalen für den Abfrageliganden, wobei die Merkmale für den Abfrageliganden einen Fingerabdruck des Liganden und einen Deskriptor des Liganden umfassen; und
Anwenden des Modells für maschinelles Lernen auf die Merkmale des Abfrageproteins und die Merkmale des Abfrageliganden, um eine Wahrscheinlichkeit einer Interaktion zwischen dem Abfrageliganden und dem Abfrageprotein zu erhalten;
wobei optional die Merkmale für das Abfrageprotein weiter mindestens eines umfassen, das ausgewählt ist aus der Gruppe bestehend aus globalen Merkmalen und funktionellen Annotationen.

10. Nicht-flüchtiges, computerlesbares Medium, das computerlesbaren Programmcode zum Vorhersagen einer Wirkstoff-Ziel-Bindung unter Verwendung synthetisch erweiterter Daten umfasst, wobei der computerlesbare Programmcode ein Computersystem veranlasst zum:
Erzeugen einer Mehrzahl von Ghost-Liganden für eine Mehrzahl von Proteinen in einer Proteinstrukturdatenbank,
wobei das Erzeugen der Mehrzahl von Ghost-Liganden umfasst:
für einen Cluster von Proteinen, der aus der Mehrzahl von Proteinen ausgewählt ist: Durchführen eines Strukturalignments für die Proteine in dem Proteincluster;
Erhalten der Mehrzahl von Ghost-Liganden durch Projizieren eines Liganden von einem der Proteine in dem Cluster auf alle anderen Proteine in dem Cluster nach dem Strukturalignment;
Erhalten, für jeden der Mehrzahl von Ghost-Liganden, eines Konfidenzwerts;
Erzeugen einer Mehrzahl von Merkmalen einer Wirkstoff-Ziel-Interaktion (DTI) für Proteine und Liganden in einer DTI-Datenbank unter Verwendung der Mehrzahl von Ghost-Liganden;
Erzeugen eines Modells für maschinelles Lernen unter Verwendung der DTI-Merkmale, wobei das Erzeugen des Modells für maschinelles Lernen umfasst:
Erhalten positiver Trainingsbeispiele auf der Grundlage der Mehrzahl von DTI-Merkmalen für die Proteine und die Liganden;
Erhalten negativer Trainingsbeispiele auf der Grundlage der Mehrzahl von DTI-Merkmalen durch mindestens einmaliges Durchmischen der Mehrzahl von DTI-Merkmalen für die Proteine und die Liganden;
unter Verwendung der positiven und der negativen Trainingsbeispiele, Trainieren des Modells für maschinelles Lernen für die DTI-Vorhersage; und
Vorhersagen einer Wahrscheinlichkeit einer Interaktion für eine Kombination aus einem Abfrageprotein und einem Abfrageliganden unter Verwendung des Modells für maschinelles Lernen.

11. System zur differenziellen Wirkstoffentdeckung, wobei das System umfasst:
eine Proteinstrukturdatenbank;
eine Engine zur Identifizierung von Ghost-Liganden, konfiguriert zum Erzeugen einer Mehrzahl von Ghost-Liganden für eine Mehrzahl von Proteinen in der Proteinstrukturdatenbank, wobei das Erzeugen der Mehrzahl von Ghost-Liganden umfasst:
für einen Cluster von Proteinen, der aus der Mehrzahl von Proteinen ausgewählt ist: Durchführen eines Strukturalignments für die Proteine in dem Proteincluster;
Erhalten der Mehrzahl von Ghost-Liganden durch Projizieren eines Liganden von einem der Proteine in dem Cluster auf alle anderen Proteine in dem Cluster nach dem Strukturalignment;
Erhalten, für jeden der Mehrzahl von Ghost-Liganden, eines Konfidenzwerts;
eine Ghost-Liganden-Datenbank zum Speichern der Mehrzahl von Ghost-Liganden;
eine Datenbank für Wirkstoff-Ziel-Interaktionen (DTI), in der Proteine und Liganden gespeichert sind;
eine Engine zur Merkmalerzeugung, konfiguriert zum Erzeugen einer Mehrzahl von DTI-Merkmalen für die Proteine und die Liganden in der DTI-Datenbank unter Verwendung der Mehrzahl von Ghost-Liganden in der Ghost-Liganden-Datenbank;
eine Engine zum Trainieren von Modellen für maschinelles Lernen, konfiguriert zum Erzeugen eines Modells für maschinelles Lernen unter Verwendung der DTI-Merkmale, wobei das Erzeugen des Modells für maschinelles Lernen umfasst:
Erhalten positiver Trainingsbeispiele auf der Grundlage der Mehrzahl von DTI-Merkmalen für die Proteine und die Liganden;
Erhalten negativer Trainingsbeispiele auf der Grundlage der Mehrzahl von DTI-Merkmalen durch mindestens einmaliges Durchmischen der Mehrzahl von DTI-Merkmalen für die Proteine und die Liganden;
unter Verwendung der positiven und der negativen Trainingsbeispiele, Trainieren des Modells für maschinelles Lernen für die DTI-Vorhersage; und
eine Engine zur DTI-Vorhersage, konfiguriert zum Vorhersagen einer Wahrscheinlichkeit einer Interaktion für eine Kombination aus einem Abfrageprotein und einem Abfrageliganden unter Verwendung des Modells für maschinelles Lernen.

12. System nach Anspruch 11, wobei das Erzeugen der Mehrzahl von DTI-Merkmalen umfasst:
für jede einer Mehrzahl von Kombinationen aus einem Liganden und einem Protein in der DTI-Datenbank:
Auswählen, aus der Mehrzahl von Ghost-Liganden, eines Ghost-Liganden, der dem für die Kombination in Betracht gezogenen Liganden am ähnlichsten ist; und
Erzeugen von Merkmalen für das für die Kombination in Betracht gezogene Protein, wobei die erzeugten Merkmale das für die Kombination in Betracht gezogene Protein charakterisieren.

13. System nach Anspruch 12, wobei die erzeugten Merkmale eines umfassen, das ausgewählt ist aus der Gruppe bestehend aus:
mindestens einem lokalen Merkmal, das Bindungsstellenmerkmale in konzentrischen Schichten mit zunehmendem Radius umfasst,
mindestens einem globalen Merkmal zusätzlich zu den Bindungsstellenmerkmalen, und
mindestens einer funktionellen Annotation;
wobei optional das Erzeugen der Mehrzahl von DTI-Merkmalen weiter umfasst:
Erhalten eines Konfidenzvektors, der eine Konfidenz in einer Mehrzahl von Komponenten der DTI-Merkmale darstellt, die dem ähnlichsten Ghost-Liganden zugeordnet sind,
wobei die Konfidenz in der Mehrzahl von Komponenten mindestens einen umfasst, der ausgewählt ist aus der Gruppe bestehend aus:
einem ersten Konfidenzwert, der eine Unsicherheit quantifiziert, die dem ähnlichsten Ghost-Liganden zugeordnet ist,
einem zweiten Konfidenzwert, der eine Ähnlichkeit der Fingerabdrücke zwischen dem für die Kombination in Betracht gezogenen Liganden und dem ähnlichsten Ghost-Liganden quantifiziert, und
einem dritten Konfidenzwert, der von der Quelle abhängig ist, aus der die DTI-Merkmale erhalten werden.

14. System nach Anspruch 11, wobei das Vorhersagen der Wahrscheinlichkeit einer Interaktion für die Kombination aus dem Abfrageprotein und dem Abfrageliganden umfasst:
Erhalten, für das Abfrageprotein, einer möglichen Bindungsstelle und zugehöriger lokaler Merkmale auf der Grundlage der Mehrzahl von Ghost-Liganden;
Erzeugen von Merkmalen für das Abfrageprotein, wobei die Merkmale für das Abfrageprotein die lokalen Merkmale umfassen;
Erzeugen von Merkmalen für den Abfrageliganden, wobei die Merkmale für den Abfrageliganden einen Fingerabdruck des Liganden und einen Deskriptor des Liganden umfassen; und
Anwenden des Modells für maschinelles Lernen auf die Merkmale des Abfrageproteins und die Merkmale des Abfrageliganden, um eine Wahrscheinlichkeit einer Interaktion zwischen dem Abfrageliganden und dem Abfrageprotein zu erhalten.

## Revendications

1. Procédé mis en œuvre par ordinateur pour prédire une liaison médicament-cible à l'aide de données augmentées de manière synthétique, le procédé comprenant :
la génération d'une pluralité de ligands fantômes pour une pluralité de protéines dans une base de données de structures protéiques,
dans lequel la génération de la pluralité de ligands fantômes comprend :
pour un amas de protéines, choisi parmi la pluralité de protéines :
la réalisation d'un alignement de structures pour les protéines de l'amas de protéines ;
l'obtention de la pluralité de ligands fantômes en projetant un ligand de l'une des protéines de l'amas sur toutes les autres protéines de l'amas, après l'alignement de structures ;
l'obtention, pour chacun de la pluralité de ligands fantômes, d'un score de confiance ;
la génération d'une pluralité de caractéristiques d'interaction médicament-cible (DTI) pour les protéines et les ligands dans une base de données DTI, à l'aide de la pluralité de ligands fantômes ;
la génération d'un modèle d'apprentissage automatique à l'aide de la pluralité de caractéristiques DTI, dans lequel la génération du modèle d'apprentissage automatique comprend :
l'obtention d'échantillons d'entraînement positifs sur la base de la pluralité de caractéristiques DTI pour les protéines et les ligands ;
l'obtention d'échantillons d'entraînement négatifs sur la base de la pluralité de caractéristiques DTI en brassant, au moins une fois, la pluralité de caractéristiques DTI pour les protéines et les ligands ;
à l'aide des échantillons d'entraînement positifs et négatifs, l'entraînement du modèle d'apprentissage automatique pour la prédiction ***DTI*** ; et
la prédiction d'une probabilité d'interaction pour une combinaison d'une protéine de requête et d'un ligand de requête à l'aide du modèle d'apprentissage automatique.

2. Procédé de la revendication 1, dans lequel l'amas de protéines est obtenu sur la base d'un élément choisi dans le groupe constitué par :
une similarité de séquence,
une similarité de topologie tridimensionnelle, et
une formation d'amas existante dans une base de données.

3. Procédé de la revendication 1, dans lequel le score de confiance quantifie une incertitude du ligand fantôme associé.

4. Procédé de la revendication 1, dans lequel la génération de la pluralité de caractéristiques DTI comprend :
pour chacune d'une pluralité de combinaisons d'un ligand et d'une protéine dans la base de données DTI :
la sélection, parmi la pluralité de ligands fantômes, d'un ligand fantôme qui est le plus similaire au ligand considéré pour la combinaison ;
éventuellement, dans lequel la sélection du ligand fantôme le plus similaire est effectuée sur la base d'une métrique de distance ; et
la génération de caractéristiques pour la protéine considérée pour la combinaison, dans lesquelles les caractéristiques générées caractérisant la protéine considérée pour la combinaison.

5. Procédé de la revendication 4, dans lequel les caractéristiques générées comprennent une caractéristique choisie dans le groupe constitué par :
au moins une caractéristique locale comprenant des caractéristiques de site de liaison dans des coquilles concentriques de rayons croissants,
au moins une caractéristique globale au-delà des caractéristiques de site de liaison, et
au moins une annotation fonctionnelle.

6. Procédé de la revendication 4, dans lequel la génération de la pluralité de caractéristiques DTI comprend en outre :
l'obtention d'un vecteur de confiance représentant une confiance dans une pluralité de composantes des caractéristiques DTI associées au ligand fantôme le plus similaire ;
éventuellement, dans lequel la confiance dans la pluralité de composantes comprend au moins un score choisi dans le groupe constitué par :
un premier score de confiance quantifiant une incertitude associée au ligand fantôme le plus similaire,
un deuxième score de confiance quantifiant une similarité d'empreinte moléculaire entre le ligand considéré pour la combinaison et le ligand fantôme le plus similaire, et
un troisième score de confiance dépendant de la source à partir de laquelle les caractéristiques DTI sont obtenues.

7. Procédé de la revendication 1, dans lequel la génération du modèle d'apprentissage automatique comprend,
avant l'obtention des échantillons d'entraînement positifs et des échantillons d'entraînement négatifs :
le filtrage de la pluralité de caractéristiques DTI pour les protéines et les ligands à l'aide d'un seuil de confiance appliqué aux vecteurs de confiance associés à la pluralité de caractéristiques DTI.

8. Procédé de la revendication 1, dans lequel le modèle d'apprentissage automatique est un modèle choisi dans le groupe constitué par un modèle de classification et un modèle de régression.

9. Procédé de la revendication 1, dans lequel la prédiction de la probabilité d'interaction pour la combinaison de la protéine de requête et du ligand de requête comprend :
l'obtention, pour la protéine de requête, d'un site de liaison possible et de caractéristiques locales associées sur la base de la pluralité de ligands fantômes ;
la génération de caractéristiques pour la protéine de requête, les caractéristiques pour la protéine de requête comprenant les caractéristiques locales ;
la génération de caractéristiques pour le ligand de requête, les caractéristiques pour le ligand de requête comprenant une empreinte moléculaire de ligand et un descripteur de ligand ; et
l'application du modèle d'apprentissage automatique aux caractéristiques de la protéine de requête et aux caractéristiques du ligand de requête afin d'obtenir une probabilité d'interaction entre le ligand de requête et la protéine de requête ;
éventuellement, dans lequel les caractéristiques pour la protéine de requête comprennent en outre au moins un élément choisi dans le groupe constitué par des caractéristiques globales et des annotations fonctionnelles.

10. Support non transitoire lisible par ordinateur comprenant un code de programme lisible par ordinateur pour prédire une liaison médicament-cible à l'aide de données augmentées de manière synthétique, le code de programme lisible par ordinateur amenant un système informatique à :
générer une pluralité de ligands fantômes pour une pluralité de protéines dans une base de données de structures protéiques,
dans lequel la génération de la pluralité de ligands fantômes comprend :
pour un amas de protéines, choisi parmi la pluralité de protéines :
la réalisation d'un alignement de structures pour les protéines de l'amas de protéines ;
l'obtention de la pluralité de ligands fantômes en projetant un ligand de l'une des protéines de l'amas sur toutes les autres protéines de l'amas, après l'alignement de structures ;
l'obtention, pour chacun de la pluralité de ligands fantômes, d'un score de confiance ;
générer une pluralité de caractéristiques d'interaction médicament-cible (DTI) pour les protéines et les ligands dans une base de données DTI, à l'aide de la pluralité de ligands fantômes ;
générer un modèle d'apprentissage automatique à l'aide de la pluralité de caractéristiques DTI, dans lequel la génération du modèle d'apprentissage automatique comprend :
l'obtention d'échantillons d'entraînement positifs sur la base de la pluralité de caractéristiques DTI pour les protéines et les ligands ;
l'obtention d'échantillons d'entraînement négatifs sur la base de la pluralité de caractéristiques DTI en brassant, au moins une fois, la pluralité de caractéristiques DTI pour les protéines et les ligands ;
à l'aide des échantillons d'entraînement positifs et négatifs, l'entraînement du modèle d'apprentissage automatique pour la prédiction DTI ; et
prédire une probabilité d'interaction pour une combinaison d'une protéine de requête et d'un ligand de requête à l'aide du modèle d'apprentissage automatique.

11. Système de découverte différentielle de médicaments, le système comprenant :
une base de données de structures protéiques ;
un moteur d'identification de ligands fantômes configuré pour générer une pluralité de ligands fantômes pour une pluralité de protéines dans la base de données de structures protéiques,
dans lequel la génération de la pluralité de ligands fantômes comprend :
pour un amas de protéines, choisi parmi la pluralité de protéines :
la réalisation d'un alignement de structures pour les protéines de l'amas de protéines ;
l'obtention de la pluralité de ligands fantômes en projetant un ligand de l'une des protéines de l'amas sur toutes les autres protéines de l'amas, après l'alignement de structures ;
l'obtention, pour chacun de la pluralité de ligands fantômes, d'un score de confiance ;
une base de données de ligands fantômes stockant la pluralité de ligands fantômes ;
une base de données d'interactions médicament-cible (DTI) stockant des protéines et des ligands ;
un moteur de génération de caractéristiques configuré pour générer une pluralité de caractéristiques DTI pour les protéines et les ligands de la base de données DTI, à l'aide de la pluralité de ligands fantômes de la base de données de ligands fantômes ;
un moteur d'entraînement de modèle d'apprentissage automatique configuré pour générer un modèle d'apprentissage automatique à l'aide des caractéristiques DTI, dans lequel la génération du modèle d'apprentissage automatique comprend :
l'obtention d'échantillons d'entraînement positifs sur la base de la pluralité de caractéristiques DTI pour les protéines et les ligands ;
l'obtention d'échantillons d'entraînement négatifs sur la base de la pluralité de caractéristiques DTI en brassant, au moins une fois, la pluralité de caractéristiques DTI pour les protéines et les ligands ;
à l'aide des échantillons d'entraînement positifs et négatifs, l'entraînement du modèle d'apprentissage automatique pour la prédiction DTI ; et
un moteur de prédiction DTI configuré pour prédire une probabilité d'interaction pour une combinaison d'une protéine de requête et d'un ligand de requête à l'aide du modèle d'apprentissage automatique.

12. Système de la revendication 11, dans lequel la génération de la pluralité de caractéristiques DTI comprend :
pour chacune d'une pluralité de combinaisons d'un ligand et d'une protéine dans la base de données DTI :
la sélection, parmi la pluralité de ligands fantômes, d'un ligand fantôme qui est le plus similaire au ligand considéré pour la combinaison ; et
la génération de caractéristiques pour la protéine considérée pour la combinaison, dans lesquelles les caractéristiques générées caractérisant la protéine considérée pour la combinaison.

13. Système de la revendication 12, dans lequel les caractéristiques générées comprennent une caractéristique choisie dans le groupe constitué par :
au moins une caractéristique locale comprenant des caractéristiques de site de liaison dans des coquilles concentriques de rayons croissants,
au moins une caractéristique globale au-delà des caractéristiques de site de liaison, et
au moins une annotation fonctionnelle.
éventuellement, dans lequel la génération de la pluralité de caractéristiques DTI comprend en outre :
l'obtention d'un vecteur de confiance représentant une confiance dans une pluralité de composantes des caractéristiques DTI associées au ligand fantôme le plus similaire ;
dans lequel la confiance dans la pluralité de composantes comprend au moins un score choisi dans le groupe constitué par :
un premier score de confiance quantifiant une incertitude associée au ligand fantôme le plus similaire,
un deuxième score de confiance quantifiant une similarité d'empreinte moléculaire entre le ligand considéré pour la combinaison et le ligand fantôme le plus similaire, et
un troisième score de confiance dépendant de la source à partir de laquelle les caractéristiques DTI sont obtenues.

14. Système de la revendication 11, dans lequel la prédiction de la probabilité d'interaction pour la combinaison de la protéine de requête et du ligand de requête comprend :
l'obtention, pour la protéine de requête, d'un site de liaison possible et de caractéristiques locales associées sur la base de la pluralité de ligands fantômes ;
la génération de caractéristiques pour la protéine de requête, les caractéristiques pour la protéine de requête comprenant les caractéristiques locales ;
la génération de caractéristiques pour le ligand de requête, les caractéristiques pour le ligand de requête comprenant une empreinte moléculaire de ligand et un descripteur de ligand ; et
l'application du modèle d'apprentissage automatique aux caractéristiques de la protéine de requête et aux caractéristiques du ligand de requête afin d'obtenir une probabilité d'interaction entre le ligand de requête et la protéine de requête.
